(19) Europäisches Patentamt
European Patent Office
Office européen des brevets

(11) Publication number: **0 376 955 B1**

(12) # EUROPEAN PATENT SPECIFICATION

(45) Date of publication of patent specification: **05.01.94**

(51) Int. Cl.[5]: **C07C 43/196**, C07D 317/22, C07D 307/00, C07F 9/117, C07F 9/177

(21) Application number: **88905940.8**

(22) Date of filing: **04.07.88**

(86) International application number: **PCT/GB88/00520**

(87) International publication number: **WO 89/00156 (12.01.89 89/02)**

Divisional application 93109760.4 filed on 04/07/88.

(54) **PREPARATION OF ISOMERS OF MYO-INOSITOL DERIVATIVES.**

(30) Priority: **02.07.87 GB 8715558**

(43) Date of publication of application: **11.07.90 Bulletin 90/28**

(45) Publication of the grant of the patent: **05.01.94 Bulletin 94/01**

(84) Designated Contracting States: **BE DE FR GB IT NL**

(56) References cited:
**EP-A- 0 269 105**

**JOURNAL OF THE CHEMICAL SOCIETY, Perkin Transactions 1, 1987, J. GIGG et al: "The allyl group for protection in carbohydrate chemistry. Part 18. Allyl and benzyl ethers of myo-inositol. Intermediates for the synthesis of myo-inositol trisphosphates", pages 423-425**

(73) Proprietor: **3i RESEARCH EXPLOITATION LIMITED**
**91 Waterloo Road**
**London SE1 8XP(GB)**

(72) Inventor: **GIGG, R H National Institute for Medical Research**
**The Ridgeway**
**Mill Hill**
**London NW7 1AA(GB)**

(74) Representative: **Eyles, Christopher Thomas et al**
**W.P. THOMPSON & CO.**
**High Holborn House**
**52-54 High Holborn**
**London WC1V 6RY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 376 955 B1

Rank Xerox (UK) Business Services
(3.10/3.9/3.3.3)

**TETRAHEDRON LETTERS**, volume 27, no. 27, 1986, Pergamon Journals Ltd., GB; S. OZAKI et al: "Total synthesis of optically active myo-inositol 1,4,5-tris(phosphate)", pp. 3157-3160

**CHEMICAL ABSTRACTS**, vol. 78, no. 7, 19 February 1973, Columbus, Ohio, US; A. E. STEPANOV et al: "Glycosylation of myo-inositol at position 2", pages 527-528, abstract no. 43895y

**JOURNAL OF THE AMERICAN CHEMICAL SOCIETY**, vol. 109, no. 11, 27 May 1987, The American Chemical Society, US; J. P. VACCA et al: "Total synthesis of D- and L-myo-inositol 1,4,5-trisphosphate", pages 3478-3479

## Description

This invention relates to intermediates for use in the synthesis of phosphate esters of inositol. Particularly, but not exclusively, the invention relates to a method which permits selective preparation of particular isomers of the numerous possible isomers of the phosphate esters which are known to exist and are created uncontrollably in some synthetic methods.

Inositol ($C_6H_6(OH)_6$, hexahydroxycyclohexane) exists in nine isomeric forms, of which myo-inositol (also known as meso-inositol and i-inositol) is of most interest. However, it will be apparent that the general principles utilised in the present invention for controlling the formation of derivatives of selected isomeric forms are applicable to other inositol isomers and, indeed, to cyclitols in general. In the interests of clarity it has to be mentioned that there has been a considerable amount of confusion in the literature in respect of the nomenclature of the cyclitols, of which group of compounds myo-inositol is a member. Reference is directed to "Cyclitol Confusion", Bernard W. Agranoff: Trends in Biosciences, 1973, 3, N 283.

Myo-inositol has the structure:

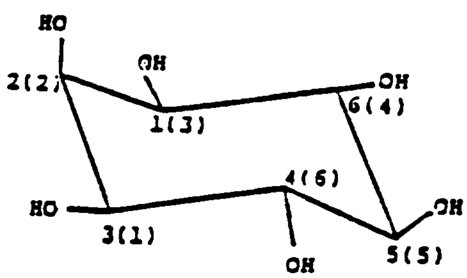

myo-INOSITOL

In this representation of the myo-inositol molecule the ring numbering is anti-clockwise, the bracketed numbers showing the clockwise numbering sequence. In myo-inositol, five of the six hydroxyl groups are of equatorial orientation and only the one at the 2-position is in axial orientation. Although myo-inositol is not itself optically active many of its derivatives are.

Certain of the inositol phosphates are known and have been isolated, with difficulty and in low yield, from natural sources. One well-known derivative of commercial importance is phytic acid (inositol hexaphosphoric acid) and its sodium salts. Also known are D-myo-inositol- 1,3,4-trisphosphate, the 1,4,5-isomer and the 1,4,5,6- and 1,3,4,5-tetrakisphosphates. These molecules are of biological importance although the precise biological role of each has not yet been fully elucidated.

Synthesis of the 1,4,5-trisphosphate has been achieved, but not entirely satisfactorily, by phosphorylation using pentavalent phosphorylating agents often resulting in the formation of undesirable five-membered phosphate rings. A description of the total synthesis of optically active myo-inositol 1,4,5-tris(phosphate) appeared in Tetrahedron Letters, Vol. 27, pages 3157 to 3160 (1986).

One major problem with all the known synthetic methods for the inositol phosphates is the uncontrollability of isomerisation. As a general rule, the biological activity of molecules is highly sensitive to their isomeric forms. It is not an uncommon occurrence, for example, for one of a pair of mirror-image isomers to be biologically active and the other to be inactive or of greatly reduced activity. Similar considerations apply to positional isomers of poly-substituted ring structures. Compounding the problem of the synthetic methods for preparation of the inositol phosphates is the fact that many of the various isomeric mixtures which are formed in the methods are, to all intents and purposes, inseparable and, in addition, it is not infrequently the case that reaction kinetics favour the formation of isomers other than those of particular interest.

Previously proposed methods for the synthesis of the inositol phosphate esters have followed the general principle of protecting, by benzylation for example, those hydroxyl groups of the inositol molecule which are to remain in unesterified form in the final product. The selectively protected molecule is then subjected to phosphorylation to introduce phosphate esters groups into those unprotected positions with free hydroxyls, and finally the molecule is deprotected. Problems are associated with these procedures in a number of respects. Firstly, insertion of the desired number and positioning of the protecting groups in the parent molecule of inositol is difficult. Secondly, the use of pentavalent phosphorylating agents has a tendency to lead to the formation of undesirable five-membered ring structures, and, finally, numerous isomeric forms arise during the synthesis which, for all practical purposes, are inseparable.

Biologically, inositol phosphates are implicated in transmembrane calcium ion transport mechanisms. For example, D-myo-inositol-1,4,5-trisphosphate is known to be generated by hydrolysis from the membrane lipid phosphatidylinositol-4,5-bisphosphate and to act as the probable second messenger for calcium mobilisation. Other esters, for example, the 1,3,4-tris- and the 1,3,4,5-tetrakis- phosphates have been isolated from stimulated cells but their precise biological roles remain as yet unclear. Suggested schemes for inositol phosphate metabolism involve, in addition to inositol itself, the mono-, bis-, tris-, and tetrakis-phosphates in several isomeric configurations.

The biological significance of inositol phosphates is the subject of much current interest and research. A need exists, then, for a method of synthesising materials for conducting scientific research and, of course, such materials are also of interest as pharmaceutical agents for therapy of clinical conditions arising from malfunction of the inositol phosphate metabolism.

Use of allyl groups for protection in the production of intermediates for the synthesis of myo-inositol trisphosphates has been described by the present inventor and his colleagues in a paper published in J. Chem. Soc. Perkin Trans. I (1987) at pages 423 to 429. Compounds (59) and (61) disclosed therein are 1,2,3,4-tetrabenzyl-6-and -5-prop-1'-enyl myo-inositol which are described as crystalline. Separation of these compounds by chromatography is also described.

British Published Patent Application No. 2198135A describes a large number of derivatives of myo-inositol or configurational isomers thereof. The derivatives are defined as cyclohexanes which are substituted with six groups ($R_1$ to $R_6$), of which three ($R_4$, $R_5$ and $R_6$) may be phosphate groups. The document states (page 5, final line to page 6, line 1) that it is not concerned with inositol triphosphate per se. Additionally, the document does not concern itself with the placing of the phosphate groups in any particular relative positions in the cyclohexane ring. It is clear that the presence of phosphate groups in the target molecule necessitates the presence of such groups in the starting material from which the derivatives are obtained. It would appear that British Published Patent Application No. 2,198,135 is concerned principally with the introduction of the three substituents $R_1$, $R_2$ and $R_3$ into an inositol which already contains the substituents $R_4$, $R_5$ and $R_6$ and which are carried unchanged from the starting material to the final product. In practical terms, then, the application of British Published Patent Application No. 2,198,135 is restricted to the derivatives of pre-existing inositol phosphates of which sufficient quantities are available for conversion to the derivatives. In common with all previously proposed reactions involving the inositol phosphates there is no indication as to how particular positional isomers of the inositol phosphates may be selectively produced - the isomers obtained by British Published Patent Application No. 2,198,135 are predestined by the isomeric form of the starting material.

An object of the present invention is to obviate or mitigate the aforesaid disadvantages.

According to the present invention there is provided a derivative of myo-inositol having the general chemical formula:

$$CyC_6H_6\ (OH)_n\ (OZ)_m\ (OX)_p \quad (A)$$

in which;

$CyC_6H_6$ represents a cyclohexane ring structure;

X is a mono- or di-valent hydroxyl- protecting group selected from benzyl, p-methoxybenzyl, crotyl and isopropylidenyl groups;

Z is a prop-1-enyl group;

n is at least 1;

m is at least 1; and

either (i) p is zero or an integer from 1 to 4 and $n+m+p=6$ when X is monovalent or (ii) p is zero, 1 or 2 and $n+m+2p=6$ when X is divalent provided that, when X is benzyl, p is not more than 3.

The invention further provides an ester of the derivative defined above with (-)-camphanic acid.

Further according to the invention there is provided a method of preparing a partially protected myo-inositol derivative of formula (A) comprising subjecting a starting material selected from myo-inositol and a myo-inositol derivative having at least one free hydroxyl group, to one or more of reactions selected from:

(a) alkylation with dibutyltin oxide in the presence of a catalyst followed by reaction of the product with an alkyl, alkenyl or aralkyl halide;

(b) alkylation with an alkyl, alkenyl or aralkyl halide in the presence of an alkali metal hydride; and,

(c) hydrolysis; and,

(d) reaction with dimethoxy propane under acid catalysts thereby to bridge one or more vicinal pairs of hydroxyl groups with an isopropylidenyl group;

including at least allylation with allyl halide to allylate a free hydroxyl group in the compound and subsequent isomerization of the resulting allyl ether group or groups to form a corresponding prop-1-enyl group or groups.

Preferably the aralkyl halide is benzyl or p-methoxybenzyl halide.

Preferably also the starting material is myo-inositol.

The principal motivation for the present invention is to permit the preparation of starting materials which have known structure for use as intermediates in the preparation of a phosphate or phosphorothioate ester of myo-inositol which may be achieved by a method comprising phosphorylating or thiophosphorylating a myo-inositol derivative of this invention and hydrolysing the phosphate or phosphorothioate derivative formed to remove the protecting group or groups.

Preferably also an allyl derivative, formed by alkylation using allyl halide, is isomerised to the corresponding prop-1-enyl derivative by treatment with potassium tert-butoxide in dimethylsulphoxide,

isomeric prop-1-enyl derivatives being then separated by chromatography.

The invention also provides a method of preparing a derivative of myo-inositol comprising reacting myo-inositol with dimethoxypropane under acid catalysis, to form a mixture of isomers of mono- and di-isopropylidenyl myo-inositol, allylating or crotylating the mixture, hydrolysing the allyl- or crotylated product to remove selectively the isopropylidenyl group or groups, reacting the hydrolysis product with dibutyltin oxide in the presence of a basic catalyst, reacting the dibutyltin complex formed with an allyl halide, reacting the allylated product with benzyl or p-methoxybenzyl halide to protect free hydroxyl groups, isomerising the allyl groups to prop-1-enyl groups, separating the prop-1-enyl derivatives, and hydrolysing each separated product to remove the prop-1-enyl groups thereby to form a myo-inositol derivative of the formula

$CyC_6H_6(OH)_x(OZ')_y$

in which $CyC_6H_6$ represents a cyclohexane ring structure;
Z' is a benzyl or p-methoxybenzyl group;
x is 3 to 5;
y is 1 to 3;
and $x + y = 6$.

Phosphate or phosphorothioate esters of myo-inositol may thereafter be prepared by phosphorylating or thiophosphorylating the free hydroxyl group or groups and hydrolysing the benzyl or p-methoxybenzyl group to deprotect the hydroxyl groups.

The present invention will now be described, by way of example, with reference to the reaction schemes set out in Figures 1 and 2 of the drawings.

In this section of the description of the invention, the myo-inositol isomers referred to are positional isomers, rather than stereoisomers, unless otherwise stated. All of the compounds I to XV are to be construed as racemic mixtures.

A mixture of compounds 2,3,6-tribenzyl-1,4-diallyl myo-inositol (formulae III, where x is a benzyl group) and 2,3,6-tribenzyl-1,5-diallyl myo-inositol (formula IV in which X is a benzyl group) were treated with potassium tert-butoxide in dimethyl sulphoxide (DMSO) to isomerise the allyl groups to prop-1-enyl groups (formulae V and VI).

The mixture of compounds V and VI was separated using column chromatography on silica gel.

Having achieved separation of the two isomers shown, the method of the invention hereafter proceeds in two separate routes. Of the various pairs of isomers involved in the reaction scheme, it is V and VI which are readily separable; separation of the other isomer pairs is extremely difficult or impossible. Thus a critical step in the method is the production of the di(prop-1-enyl) derivatives having formulae V and VI to enable separation to be achieved.

Analysis of compounds V and VI by methylation followed by acidic hydrolysis to remove the prop-1-enyl groups and hydrogenolysis to remove the benzyl groups gave the O-methyl derivatives which were characterised as the pentaacetates (well-known structures) thus establishing unequivocally the structures of V and VI.

Compounds V and VI were allylated to give the compounds of structure VII and VIII and subsequent dilute acid hydrolysis to remove the prop-1-enyl groups gave the target compounds IA and IIA (Figure 1).

The allyl groups of compounds IA and IIA may be isomerised with potassium tert-butoxide in DMSO to give the respective prop-1-enyl (IB and IIB) derivatives as analytically pure compounds which have crystalline acetates.

One intended use for the compounds I and II is in the preparation of selected isomers of inositol phosphates and phosphorothioates. In Figure 2 a reaction scheme for the preparation of the phosphates and phosphorothioates is shown.

Compound I or II having Z representing a prop-1-enyl group, is phosphorylated using the compound $(Cl_3CCH_2O)_2POCl$ in pyridine or phosphitylated and the product oxidized to give the bisphosphates IX or X (Y being oxygen, in this case) and subsequent removal of the prop-1-enyl groups by dilute acid hydrolysis or treatment with mercury (II) chloride gave the bisphosphates XI or XII (Y being oxygen) without cyclisation of the phosphotriester groups. It is to be noted that treatment with limited amounts of the phosphorylating agent $[(Cl_3CCH_2)_2POCl]$ results in formation of the respective pairs of isomers of the monophosphates which can be readily separated by chromatography.

Treatment of the phosphites from I or II with sulphur in pyridine gives the phosphorothioates IX and X (Y being sulphur, in this case).

Removal of the protecting benzyl groups and R groups from XI and XII, yields the inositol phosphates (XIII and XIV).

The compounds XI and XII (Y being oxygen or sulphur) may be further thiophosphorylated or phosphorylated to yield the trisphosphate/phosphorothioate having the formula XV in which Y is oxygen and/or sulphur.

The starting materials of this invention may be derived according to the reaction scheme set forth in Figure 3, from 2,3,6-tribenzyl-4,5-isopropylidenyl myo-inositol having the formula XVI, [a synthesis of which is described in J.Chem. Soc.; Perkin Trans.

1, (1987) 423] (Bz being benzyl).

The compound of formula XVI was converted to the allyl ether of formula XVII (All being allyl) by treatment with allyl bromide, sodium hydride and dry N,N'-dimethylformamide and subsequent acidic hydrolysis to remove the isopropylidene group.

The allyl ether XVII was converted, by standard procedures to the dibutyltin derivative having the formula XVIII (Bu being butyl) which, on reaction with allyl bromide in toluene in the presence of tetrabutylammonium bromide, yielded the isomer mixture of compounds of formulae III and IV.

Concerning now the stereochemistry of the compounds involved in this invention, the racemate of the isopropylidenyl derivative having the formula XVI may be resolved into its optically active isomers via the camphanate esters, using (-)-camphanic acid chloride to give readily and in high yield (85%) the camphanate of 1,2,4-tri-O-benzyl-5,6-O-isopropylidenyl-D-myo-inositol, the structure of which was confirmed by conversion, by acid hydrolysis followed by hydrogenolysis, into 1-O-methyl-L-myo-inositol [(+)-bornesitol] the structure of which is known. From the other liquors remaining after removal of the crystalline camphanate described above there was prepared 1-O-allyl-2,3,6-tri-O-benzyl-D-myo-inositol (XVII) in optically pure and crystalline form. Resolution allows the reaction scheme shown in Figure 3 to be carried out using the separated optical isomers as compound XVI.

The di(prop-1-enyl) derivatives of the invention may be prepared by isomerization of the corresponding diallyether. The specific advantage which the di(prop-1-enyl) diethers have over the allyl (that is, the prop-2-enyl) diethers is that it is possible to separate them by ordinary separation techniques such as chromatography whereas the allyl compounds are impossible or inordinately difficult to separate.

The most convenient source material for this invention is myo-inositol itself and this will require pretreatment in order to protect the hydroxyl groups in selected positions leaving free hydroxyls in those locations where the phosphate ester groups are to be located in the final product. This controlled substitution of myo-inositol utilizes a number of facts:

(1) In the chair form of the cyclohexane ring of myo-inositol all the hydroxy groups save one (at position-2) are in the equatorial orientation;

(2) Reaction of a polyol with dimethoxypropane results in bridging of vicinal hydroxy groups with the divalent isopropylidenyl group; and

(3) "Tin alkylation" (explained below) of an equatorial-axial pair of vicinal hydroxyls favours the monoalkylation in the equatorial position. Alkylation by this same reaction of an equatorial-equatorial pair of vicinal hydroxyls results in monoalkylation of one or other of the locations giving a mixture.

(4) Allyl, isopropylidenyl, benzyl and prop-1-enyl groups may be removed by hydrolysis under conditions which are sufficiently different from each other to enable selective removal of one group in the presence of one or more of the others.

By selective use of these facts it is possible to devise reaction schemes which, combined with separation, where convenient, of the components of mixed reaction products, will produce the necessary selectively blocked myo-inositols for use as the starting materials of this invention.

One such scheme will now be described by way of illustration, but it will be realised that other possible sequences of events can be devised to give particular end-products.

Referring to Figure 4 of the drawings herewith, a reaction scheme shown begins with the readily available myo-inositol. In the myo-inositol structure shown, it is the hydroxy group in the 2-position which is in axial orientation, the remaining five hydroxyls being equatorially bonded. In the Figures, the symbol ± inside the cyclohexane ring indicates a racemate, the absence of a symbol indicates a meso (i.e. symmetrical) compound and those rings shown with a heavily lined front edge are enantiomers (i.e. pure optical isomers).

Referring to Figure 4, myo-inositol (formula 10) is treated with dimethoxypropane under acidic catalysis using p-toluenesulphonic acid, for example, in order to bridge vicinal hydroxy groups with an isopropylidenyl group [Reaction (a)]. This reaction results in a mixture of four products: a mono-iso-propylidenyl compound (11) and three isomeric diisopropylidenyl compounds (12 to 14).

Allylation of the mixture of four compounds 11 to 14 with allyl bromide and sodium hydride (Reaction (b)] results in four products shown as formulae 15 to 18 in which the free hydroxyls of 12 to 14 have been converted to the allyl ether, that is, the tetraallylisopropylidenyl-myo-inositol 15 and three diallyldiisopropylidenyl-myo-inositol derivatives 16, 17, 18. It is possible to substitute crotyl bromide for the allyl bromide to give the crotyl ethers. Hereafter references to allyl groups should be read as including crotyl groups in the alternative.

Hydrolysis of the products 15 to 18 with acetic acid at 100°C [Reaction (c)] replaces the isopropylidenyl groups with hydroxyls, giving compounds 19 to 22, tetraallyl-myo-inositol 19 and three diallyl-myo-inositol derivatives, 20, 21, 22.

It is convenient at this point in the scheme shown to recover the tetraallyl-myo-inositol (19) by extraction with petroleum ether in which the diallyl-myo-inositols 20, 21, 22 are insoluble.

"Tin alkylation" is a convenient name for an alkylation reaction which involves reaction with dibutyltin oxide in the presence of a catalyst (for example tetrabutylammonium bromide) followed by treatment with an optionally substituted alkyl halide (allyl bromide, for example). This involves the placement, with a high degree of selectivity, of an alkyl substituent at the equatorial position of an equatorial/axial vicinal hydroxy pair (as at positions 1 and 2) and at either position of an equatorial/equatorial pair (as at positions 5 and 6 or 3 and 4)). Tin alkylation of the mixture of compounds 20 to 22 (reaction (d)) results in the insertion of allyl at position 1 of the ring, that is alkylation at equatorial bond at position-1 in preference to the axial bond at position-2, giving compounds 23 (from both 20 and 21) and compounds 24 and 25 from allylation of one or the other of the equatorial hydroxyls at position 1 (compound 24) and position 3 (compound 25) from compound 22. Further allylation inserts allyls at position 4 or 5 of compound 23 to give a mixture of tetraallyl derivatives 26 and 27, or at position 3 of compound 24, resulting in compound 27 or at position 1 of compound 25 giving, again, compound 27.

Returning to compound 19, isolated earlier, this tetraallyl-myo-inositol may also be subjected to tin alkylation in the manner described above, with the result that an allyl group enters the equatorial position 1 in preference to the axial position 2 of the vicinal pair of hydroxyls involved in the tin alkylation reaction, yielding the pentaallyl-myo-inositol of formula 28.

The allyl derivatives of myo-inositol presented by this scheme may be benzylated at the free hydroxyls. The benzylated product may then be deallylated to give free hydroxyls. This provides a compound (exemplified as compound XVI in Figure 3) for treatment by the method of this invention to compounds which are intermediates in the route to the myo-inositol phosphate and phosphorothioate esters. Thus precursors for the following compounds are available as a result of the Figure 4 scheme shown:

(a) myo-inositol-3,6-bisphosphate (from 20)
(b) myo-inositol-3,4-bisphosphate (from 21)
(c) myo-inositol-5,6-bisphosphate (from 22)
(d) myo-inositol-1,3,6-trisphosphate (from 23)
(e) myo-inositol-1,5,6-trisphosphate (from 24)
(f) myo-inositol-3,5,6-trisphosphate (from 25)
(g) myo-inositol-1,3,4,6-tetrakisphosphate (from 26)
(h) myo-inositol-1,3,5,6-tetrakisphosphate (from 27)
(i) myo-inositol-3,4,5,6-tetrakisphosphate (from 19)
(j) myo-inositol-1,3,4,5,6-pentakisphosphate (from 28)

[Note: the numbering of these compounds would normally be rationalised but the numbering quoted here is used to emphasise that the positions of the phosphate ester groups in the final product are determined by the positions of the allyls in the formulae 19 to 28 above].

It is convenient at this point in the description to discuss the resolution of those compounds shown in Figure 4 which are racemic, that is, those having the symbol ± inside the cyclohexane ring symbol. For the purpose of illustration, compound 27 (1,3,5,6-tetraallyl-myo-inositol) has been taken as an example in Figure 5.

Referring to Figure 5, 1,3,5,6-tetraallyl-myo-inositol 27 is esterified with camphanic acid to yield the biscamphanate as a pair of diastereoisomers 29 and 30 which were separated by column chromatography and crystallisation. Subsequent basic hydrolysis of the separated compounds 29 and 30 restores hydroxyls to the positions of the camphanate ester groups to give compounds 31 and 32. Compound 32 is of particular interest and its absolute configuration was established by conversion to the known optically active di-O-methyl ether 33.

Figure 6 illustrates the resolution of compound 23 (1,3,6-triallyl-myo-inositol). The sequence of reactions is as follows: treatment of compound 23 with dimethoxy propane as before, to bridge the vicinal hydroxyls at positions 4 and 5 to give compound 34, benzylation of the isopropylidenyl derivative 34 to benzylate the free hydroxyl at position 2 to give compound 35, and removal by hydrolysis of the isopropylidenyl group to free the hydroxyls at positions 4 and 5, giving the racemate 36 (2-O-benzyl-1,3,6-triallyl-myo-inositol). Formation of the biscamphanate gave compounds 37 and 38 which were separated by column chromatography and crystallisation. Removal of the camphanate ester group from compound 38 gave 39 the configuration of which was confirmed by conversion to the dimethyl diether 40.

Figure 7 shows the conversion of examples of certain resolved compounds to the benzyl ethers necessary for the introduction of the phosphate ester groups. Benzylation of 39 yielded the tri-O-benzyl-triallyl-myo-inositol 41 which, following hydrolysis to remove the allyls, yielded 42, tri-O-benzyl-myo-inositol. Compound 32, on similar treatment, yielded compound 43 which was then converted to the di-O-benzyl-myo-inositol 44. In similar fashion compound 26 gave 46 via the intermediate 45, and 28 gave 48 via 47.

Figure 8, using compound 44 as an example, illustrates the conversion to the phosphate esters. Compound 44 (2,6-di-O-benzyl-myo-inositol) is reacted with dimethoxy propane to give the pair of positional isomers 49 and 50, in which vicinal

hydroxyls are bridged by an isopropylidenyl group, which were separated by chromatography. Separately, compounds 49 and 50 were phosphorylated or thiophosphorylated to esterify the free hydroxyls at positions 1 and 5 in compound 49 and positions 1 and 3 in compound 50, the products being shown as formulae 51 and 52 in which R represents a phosphate or phosphorothioate ester. Removal of the isopropylidenyl groups from 51 and 52 frees the hydroxyls at the respective positions, giving 53 and 54, allowing further phosphorylation or thiophosphorylation of the free hydroxyls to yield compounds 55 and 56, from which the benzyl groups may be removed to yield the phosphate, phosphorothioate or mixed phosphate/phosphorothioate derivatives.

Figure 9 shows conversion of compound 19 (see Figure 4) to the compound 1,2-di-O-benzyl compound 58 via the intermediate allyated derivative 57.

Figure 10 shows the preparation, based on compound 20 as starting material, of suitable myo-inositol derivatives for conversion to the phosphate esters. Using the tin alkylation reaction previously described, compound 20 was crotylated to give 59 which was then reacted with dimethoxy propane to bridge the vicinal hydroxyls at positions 4 and 5 to give compound 60 and the single free hydroxyl at position 2 was then allylated to yield 61. Compound 61 was hydrolysed to remove the isopropylidenyl groups and free the hydroxyls at positions 4 and 5 (compound 62). Resolution of racemate 62 was achieved, as previously described, via the camphanate esters 63 and 64. Treatment of compound 63, of particular interest, with potassium tert-butoxide in dimethylsulphoxide removed the crotyl group and isomerised the allyl groups to prop-1-enyl groups (compound 66) which is a suitable intermediate on the route to the corresponding phosphate/phosphorothioate ester of myo-inositol.

It will be appreciated that there are numerous variations in the selection of reactions to which the starting materials of the invention may be subjected and in the order in which these reactions may be carried out. However, provided the structure of the starting myo-inositol compound is known, the outcome of each of the reactions may be accurately predicted from the information given herein and an appropriate sequence of reactions selected with the structure of the target derivative in mind. Esterification with (-)-camphanic acid chloride forms the camphanate esters for resolution of optical isomers.

While the present invention is principally concerned with the eventual preparation of phosphate and phosphorothioate esters of myo-inositol, it will be readily appreciated that the partially protected myo-inositol isomers which the present invention produces provide suitable starting materials for reaction via the free hydroxyl groups to myo-inositol derivatives other than the phosphates and phosphorothioates, the advantage lying, of course, in the fact that the structure of the products can be closely manipulated, by this invention, to derive specific isomeric forms.

## Claims

**1.** A derivative of myo-inositol having the general chemical formula:

$$CyC_6H_6\ (OH)_n\ (OZ)_m\ (OX)_p \qquad (A)$$

in which;
$CyC_6H_6$ represents a cyclohexane ring structure;
X is a mono- or di-valent hydroxyl- protecting group selected from benzyl, p-methoxybenzyl, crotyl and isopropylidenyl groups;
Z is a prop-1-enyl group;
n is at least 1;
m is at least 1; and
either (i) p is zero or an integer from 1 to 4 and $n+m+p=6$ when X is monovalent or (ii) p is zero, 1 or 2 and $n+m+2p=6$ when X is divalent, provided that, when X is benzyl, p is not more than 3.

**2.** An ester of the derivative claimed in claim 1 with (-)-camphanic acid.

**3.** A method of preparing a partially protected myo-inositol derivative as claimed in claim 1 comprising subjecting a starting material selected from myo-inositol and a myo-inositol derivative having at least one free hydroxyl group, to one or more of reactions selected from:
(a) alkylation with dibutyltin oxide in the presence of a catalyst followed by reaction of the product with an alkyl, alkenyl or aralkyl halide;
(b) alkylation with an alkyl, alkenyl or aralkyl halide in the presence of an alkali metal hydride; and,
(c) hydrolysis; and,
(d) reaction with dimethoxy propane under acid catalysis thereby to bridge one or more vicinal pairs of hydroxyl groups with an isopropylidenyl group;
including at least allylation with allyl halide to allylate a free hydroxyl group in the compound and subsequent isomerization of the resulting allyl ether group or groups to form a corresponding prop-1-enyl ether group or groups.

4. A method as claimed in claim 3, in which the aralkyl halide is benzyl or p-methoxybenzyl halide.

5. A method as claimed in claim 3 or claim 4 in which the starting material is myo-inositol.

6. A method as claimed in any of claims 3 to 5, in which an allyl derivative, formed by alkylation using allyl halide, is isomerised to the corresponding prop-1-enyl derivative by treatment with potassium tert-butoxide in dimethylsulphoxide.

7. A method as claimed in claim 6, in which a mixture of isomeric prop-1-enyl derivatives are separated.

8. A method as claimed in claim 7, in which said separation is effected by chromatography.

9. A method according to any one of claims 3 to 8, which includes the steps of esterifying a myo-inositol derivative having at least one free hydroxyl group with camphanic acid, separating the resulting pair of diastereoisomers by column chromatography and crystallisation, and hydrolysis of the respective separated diastereoisomer.

10. A method of preparing a phosphate or phosphorothioate ester of myo-inositol comprising phosphorylating or thiophosphorylating a myo-inositol derivative claimed in claim 1 and hydrolysing the phosphate or phosphorothioate derivative formed to remove the protecting group or groups.

11. A method of preparing a derivative of myo-inositol comprising reacting myo-inositol with dimethoxypropane under acid catalysis conditions, to form a mixture of isomers of mono- and di-isopropylidenyl myo-inositol, allylating or crotylating the mixture, hydrolysing the allyl- or crotylated product to remove selectively the isopropylidenyl group or groups, reacting the hydrolysis product with dibutyltin oxide in the presence of a catalyst, reacting the dibutyltin complex formed with an allyl halide, reacting the allylated product with benzyl or p-methoxybenzyl halide to protect free hydroxyl groups, isomerising the allyl groups to prop-1-enyl groups, separating the prop-1-enyl derivatives, and hydrolysing each separated product to remove the prop-1-enyl groups thereby to form a myo-inositol derivative of the formula

$$CyC_6H_6(OH)_x(OZ')_y$$

in which $CyC_6H_6$ represents a cyclohexane ring structure;
Z' is a benzyl or p-methoxybenzyl group;
x is 3 to 5;
y is 1 to 3;
and $x + y = 6$.

12. A method as claimed in claim 11 including the additional step of phosphorylating or thiophosphorylating the free hydroxyl group or groups and hydrolysing the benzyl or p-methoxybenzyl group to deprotect the hydroxyl group or groups.

13. A method as claimed in any one of claims 10 to 12, which includes the steps of esterifying a myo-inositol derivative having at least one free hydroxyl group with camphanic acid, separating the resulting pair of diastereoisomers by column chromatography and crystallisation, and hydrolysis of the respective separated diastereoisomer.

**Patentansprüche**

1. Derivat des myo-Inositol entsprechend der allgemeinen chemischen Formel:

$$CyC_6H_6\ (OH)_n\ (OZ)_m\ (OX)_p \qquad (A)$$

in der;
$CyC_6H_6$ eine Cyclohexan-Ringstruktur darstellt;
X eine ein- oder zweiwertige, aus der Gruppe Benzyl, p-Methoxybenzyl, Crotyl und Isopropylidenyl ausgewählte Hydroxylschutzgruppe ist;
Z eine Prop-1-enyl-Gruppe ist;
n mindestens 1 ist;
m mindestens 1 ist; und
entweder (i) p 0 oder eine ganze Zahl von 1 bis 4 ist und $n + m + p = 6$, wenn X einwertig ist oder (ii) p 0, 1 oder 2 ist und $n + m + 2p = 6$, wenn X zweiwertig ist, vorausgesetzt daß, wenn X Benzyl ist, p nicht größer als 3 ist.

2. Ein Ester des Derivats gemäß Anspruch 1 mit (-)-Camphansäure.

3. Verfahren zur Herstellung von teilweise geschütztem myo-Inositol-Derivat gemäß Anspruch 1, das ein Startmaterial, ausgewählt aus myo-Inositol und myo-Inositolderivat, welches mindestens eine freie Hydroxylgruppe aufweist, umfaßt, das einer oder mehrerer der folgenden Reaktionen unterworfen wird:
(a) Alkylierung mit Dibutylzinnoxid in Gegenwart eines Katalysators und anschließender Reaktion des Produkts mit einem Alkyl-,

Alkenyl- oder Aralkylhalogenid;

(b) Alkylierung mit einem Alkyl-, Alkenyl- oder Aralkylhalogenid in Gegenwart eines Alkalimetallhydrids; und,

(c) Hydrolyse; und

(d) Reaktion mit Dimethoxypropan mittels Säurekatalyse unter Verbrückung eines oder mehrerer vicinaler Hydroxyl-Gruppenpaare mit einer Isopropylidenylgruppe;

einschließlich mindestens einer Allylierung mit Allylhalogenid, um eine freie Hydroxylgruppe in der Verbindung zu allylieren und um anschließend die resultierende Allylether-Gruppe oder -Gruppen unter Bildung der entsprechenden Prop-1-enylether-Gruppe oder -Gruppen zu isomerisieren.

**4.** Verfahren gemäß Anspruch 3, in dem das Aralkylhalogenid ein Benzyl- oder p-Methoxybenzylhalogenid ist.

**5.** Verfahren gemäß Anspruch 3 oder Anspruch 4, in dem das Startmaterial myo-Inositol ist.

**6.** Verfahren gemäß einem jeden der Ansprüche 3 bis 5, in dem ein Allylderivat, gebildet durch Alkylierung unter Verwendung von Allylhalogenid, zu dem entsprechenden Prop-1-enyl-Derivat unter Behandlung mit Kalium-tert-butylat in Dimethylsulfoxid, isomerisiert wird.

**7.** Verfahren gemäß Anspruch 6, in dem eine Mischung der isomeren Prop-1-enyl-Derivate getrennt wird.

**8.** Verfahren gemäß Anspruch 7, in dem die Trennung mittels Chromatographie erfolgt.

**9.** Verfahren gemäß einem jeden der Ansprüche 3 bis 8, das die Schritte einschließt: Veresterung des myo-Inositol-Derivats, welches mindestens eine freie Hydroylgruppe aufweist mit Camphansäure, Trennung des entstehenden Diastereomerenpaars mittels Säulenchromatographie und Kristallisation, und Hydroylse des jeweiligen getrennten Diastereomeren.

**10.** Verfahren zur Herstellung eines Phosphats oder Phosphorigthioatesters des myo-Inositols, welches eine Phosphorylierung oder Thiophosphorylierung eines myo-Inositol-Derivats gemäß Anspruch 1 umfaßt sowie zur Entfernung der Schutzgruppe oder -gruppen eine Hydrolyse des Phosphats oder des gebildeten Phophorigthioat-Derivats.

**11.** Verfahren zur Herstellung eines Derivats von myo-Inositol, das die Umsetzung von myo-Inositol mit Dimethoxypropan unter säurekatalytischen Bedingungen umfaßt, unter Bildung einer Mischung der Isomeren des Mono- und des Diisopropylidenyl des myo-Inositols, Allylierung oder Crotylierung der Mischung, Hydrolyse des allyl- oder crotylierten Produkts zur selektiven Entfernung der Isopropylidenyl-Gruppe oder -Gruppen, Reaktion des Hydrolyseprodukts mit Dibutylzinnoxid in Gegenwart eines Katalysators, Reaktion des gebildeten Dibutylzinnkomplexes mit einem Allylhalogenid, Umsetzung des allylierten Produkts mit Benzyl- oder p-Methoxybenzylhalogenid, um die freien Hydroxylgruppen zu schützen, Isomerisierung der Allylgruppen zur Prop-1-enylgruppen, Trennung der Prop-1-enyl-Derivate sowie Hydrolyse jedes einzelnen getrennten Produkts, zur Entfernung der Prop-1-enylgruppen, unter Bildung eines myo-Inositol-Derivats mit der Formel

$CyC_6H_6(OH)_x(OZ')_y$

in der $CyC_6H_6$ eine Cyclohexanring-Struktur bedeutet;
Z' eine Benzyl- oder p-Methoxybenzylgruppe ist;
x 3 bis 5 ist;
y 1 bis 3 ist;
und x + y = 6 ist.

**12.** Verfahren gemäß Anspruch 11, das zusätzlich den Schritt umfaßt einer Phosphorylierung oder Thiophosphorylierung der freien Hydroxylgruppe oder -gruppen und Hydrolyse der Benzyl- oder p-Methoxybenzylgruppe, um die Hydroxylgruppe oder -gruppen zu entschützen.

**13.** Verfahren gemäß eines jeden der Ansprüche 10 bis 12, das die Schritte umfaßt: Veresterung eines myo-Inositol-Derivats, welches mindestens eine freie Hydroxyl-Gruppe aufweist, mit Camphansäure, Trennung des erhaltenden Diastereomerenpaars mittels Säulenchromatographie und Kristallisation sowie Hydrolyse des jeweiligen getrennten Diastereomeren.

## Revendications

**1.** Un dérivé de myo-inositol répondant à la formule chimique générale :

$CyC_6H_6\ (OH)_n\ (OZ)_m\ (OX)_p$ (A)

dans laquelle
$CyC_6H_6$ réprésente une structure de noyau cyclohexane ;
X est un groupe protecteur monovalent ou

divalent du groupe hydroxyle choisi parmi les groupes benzyle, p-méthoxybenzyle, crotyle et isopropylidényle ;
Z est un groupe 1-propényle ;
n est au moins égal à 1 ;
m est au moins égal à 1 ; et
ou bien (i) p est égal à 0 ou un entier de 1 à 4 et n + m + p = 6 lorsque X est monovalent, ou bien (ii) p est égal à 0, 1 ou 2 et n + m + 2p = 6 lorsque X est divalent, à condition que lorsque X est un groupe benzyle, p ne soit pas supérieur à 3.

**2.** Un ester du dérivé selon la revendication 1 avec l'acide (-)-camphanique.

**3.** Un procédé de préparation d'un dérivé de myo-inositol partiellement protégé selon la revendication 1, consistant à soumettre un produit de départ choisi parmi le myo-inositol et un dérivé de myo-inositol ayant au mois un groupe hydroxyle libre à une ou plusieurs réactions choisies parmi les suivantes :
a) alkylation par l'oxyde de dibutylétain en présence d'un catalyseur suivie de réaction du produit avec un halogénure d'alkyle, d'alcényle ou d'aralkyle ;
b) alkylation par un halogénure d'alkyle, d'alcényle ou d'aralkyle en présence d'un hydrure de métal alcalin ; et
c) hydrolyse ; et
d) réaction avec le diméthoxypropane avec catalyse acide pour relier une ou plusieurs paires de groupes hydroxyles vicinaux par pontage par un groupe isopropylidényle ;
comprenant au moins l'allylation par un halogénure d'allyle pour allyler un groupe hydroxyle libre dans le composé et l'isomérisation subséquente du ou des groupes éthers d'allyle résultants pour former un ou des groupes éthers de 1-propényle correspondants.

**4.** Un procédé selon la revendication 3, dans lequel l'halogénure d'aralkyle est l'halogénure de benzyle ou de p-méthoxybenzyle.

**5.** Un procédé selon la revendication 3 ou 4, dans lequel le produit de départ est le myo-inositol.

**6.** Un procédé selon l'une quelconque des revendications 3 à 5, dans lequel un dérivé allylé, formé par alkylation en utilisant un halogénure d'allyle, est isomérisé en le dérivé 1-propénylé correspondant par traitement par le tert-butoxyde de potassium dans le diméthylsulfoxyde.

**7.** Un procédé selon la revendication 6, dans lequel on sépare un mélange de dérivés 1-propénylés isomères.

**8.** Un procédé selon la revendication 7, dans lequel ladite séparation est effectuée par chromatographie.

**9.** Un procédé selon l'une quelconque des revendications 3 à 8, qui comprend les étapes d'estérification d'un dérivé de myo-inositol ayant au moins un groupe hydroxyle libre par l'acide camphanique, séparation de la paire de diastéréoisomères résultante par chromatographie sur colonne et cristallisation et hydrolyse du diastéréoisomère séparé respectif.

**10.** Un procédé de préparation d'un ester phosphate ou phosphorothioate de myo-inositol comprenant la phosphorylation ou thiophosphorylation d'un dérivé de myo-inositol selon la revendication 1 et l'hydrolyse du dérivé phosphate ou phosphorothioate formé pour séparer le ou les groupes protecteurs.

**11.** Un procédé de préparation d'un dérivé de myo-inositol comprenant la réaction du myo-inositol avec le diméthoxypropane dans des conditions de catalyse acide, pour former un mélange d'isomères de mono- et diisopropylidényl-myo-inositols, l'allylation ou la crotylation du mélange, l'hydrolyse du produit allylé ou crotylé pour séparer sélectivement le ou les groupes isopropylidényles, la réaction du produit d'hydrolyse avec l'oxyde de dibutylétain en présence d'un catalyseur, la réaction du complexe de dibutylétain formé avec un halogénure d'allyle, la réaction du produit allylé avec un halogénure de benzyle ou de p-méthoxybenzyle pour protéger les groupes hydroxyles libres, l'isomérisation des groupes allyles en groupes 1-propényles, la séparation des dérivés 1-propénylés et l'hydrolyse de chaque produit séparé pour séparer les groupes 1-propényles en formant ainsi un dérivé de myo-inositol de formule :

$CyC_6H_6(OH)_x(OZ')_y$

dans laquelle $CyC_6H_6$ représente une structure de noyau cyclohexane ;
Z' est un groupe benzyle ou p-méthoxybenzyle ;
x est un entier de 3 à 5 ;
y est un entier de 1 à 3 ;
et x + y = 6.

**12.** Un procédé selon la revendication 11, comprenant l'étape supplémentaire de phosphorylation ou thiophosphorylation du ou des groupes hydroxyles libres et d'hydrolyse du groupe benzyle ou p-méthoxybenzyle pour libérer le ou les groupes hydroxyles protégés.

**13.** Un procédé selon l'une quelconque des revendications 10 à 12, qui comprend les étapes d'estérification d'un dérivé de myo-inositol ayant au moins un groupe hydroxyle libre par l'acide camphanique, de séparation de la paire de diastéréoisomères résultante par chromatographie sur colonne et de cristallisation et d'hydrolyse du diastéréoisomère séparé respectif.

FIG. 1.

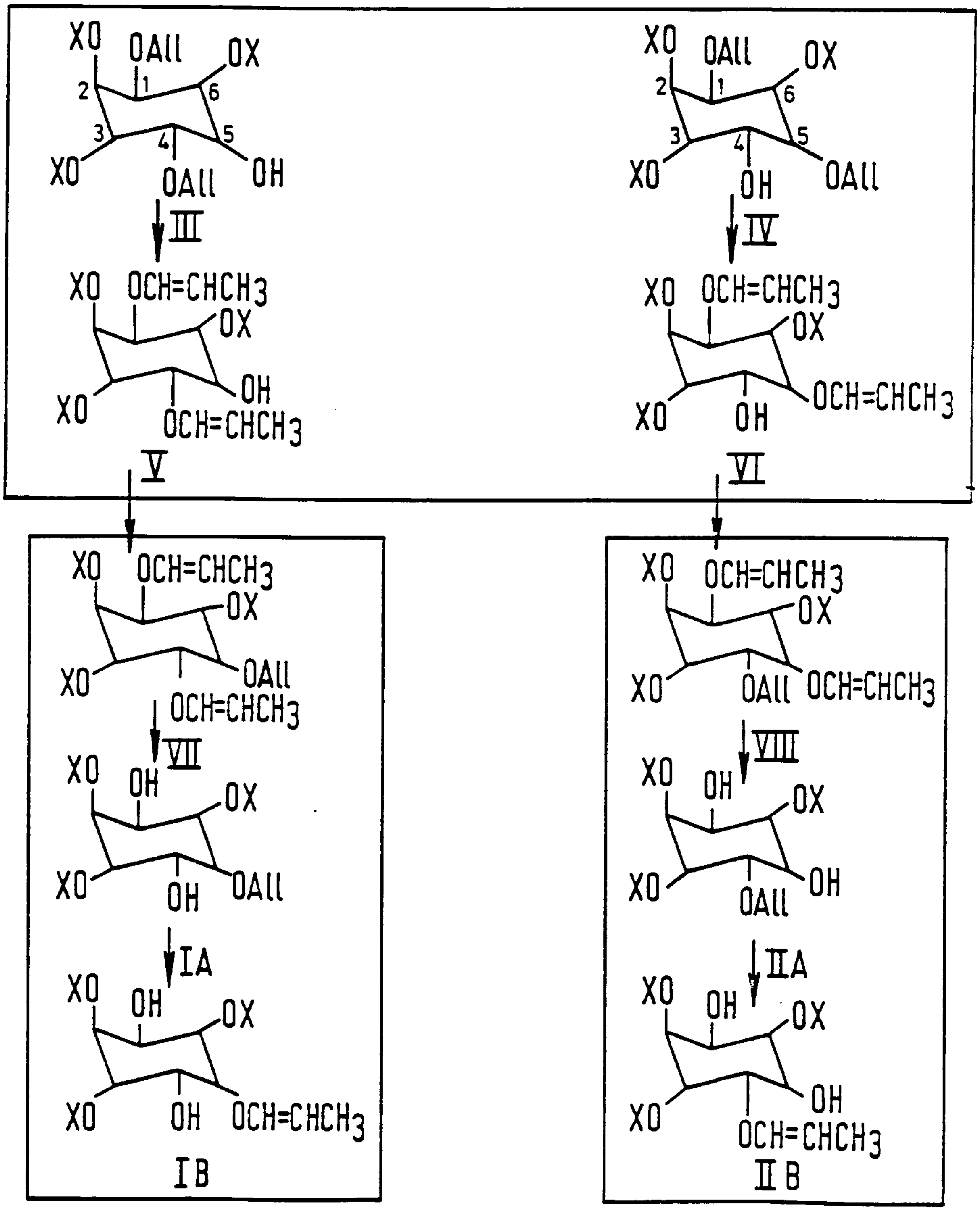

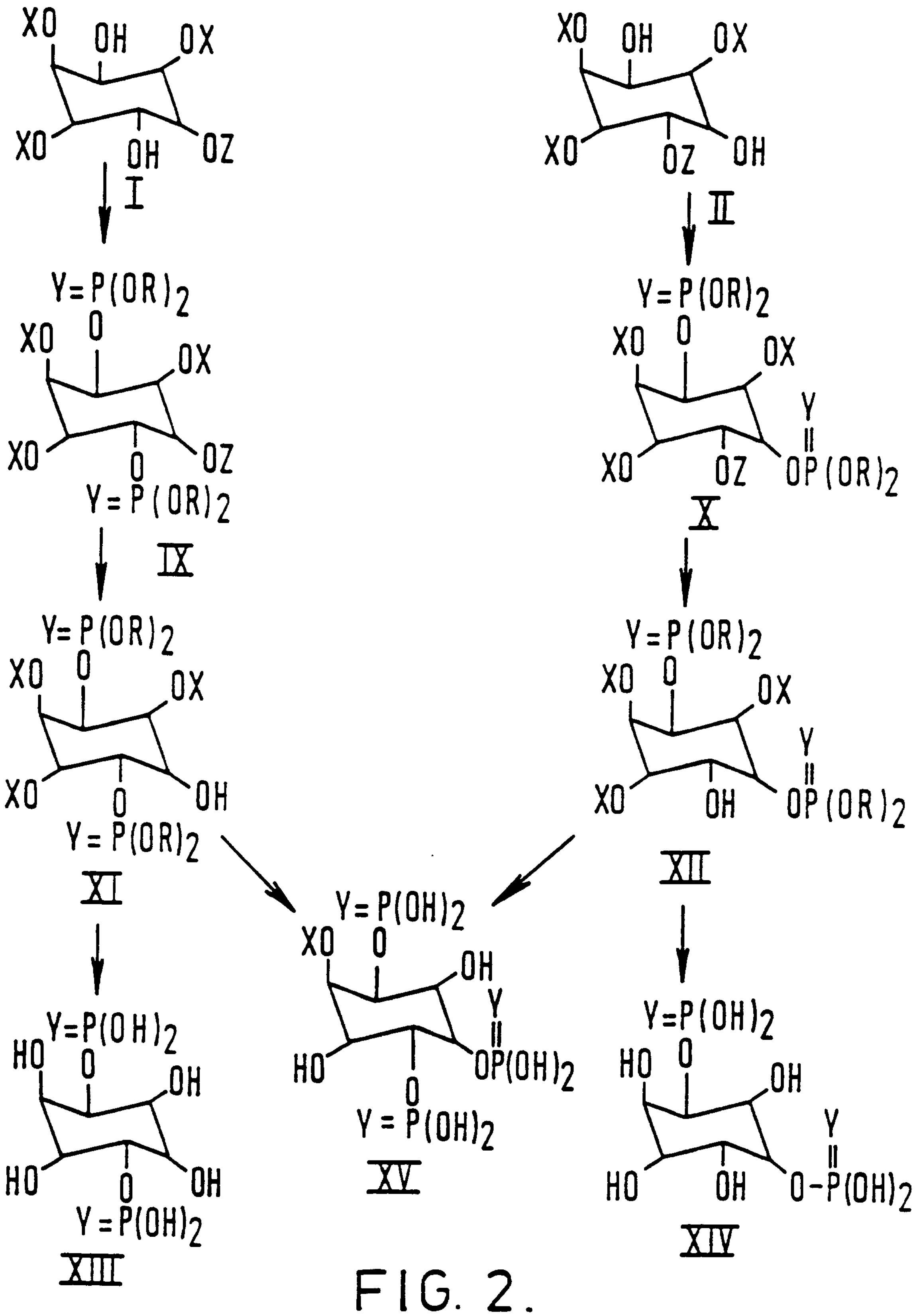

I

II

IX

X

XI

XII

XV

XIII

XIV

FIG. 2.

FIG. 3.

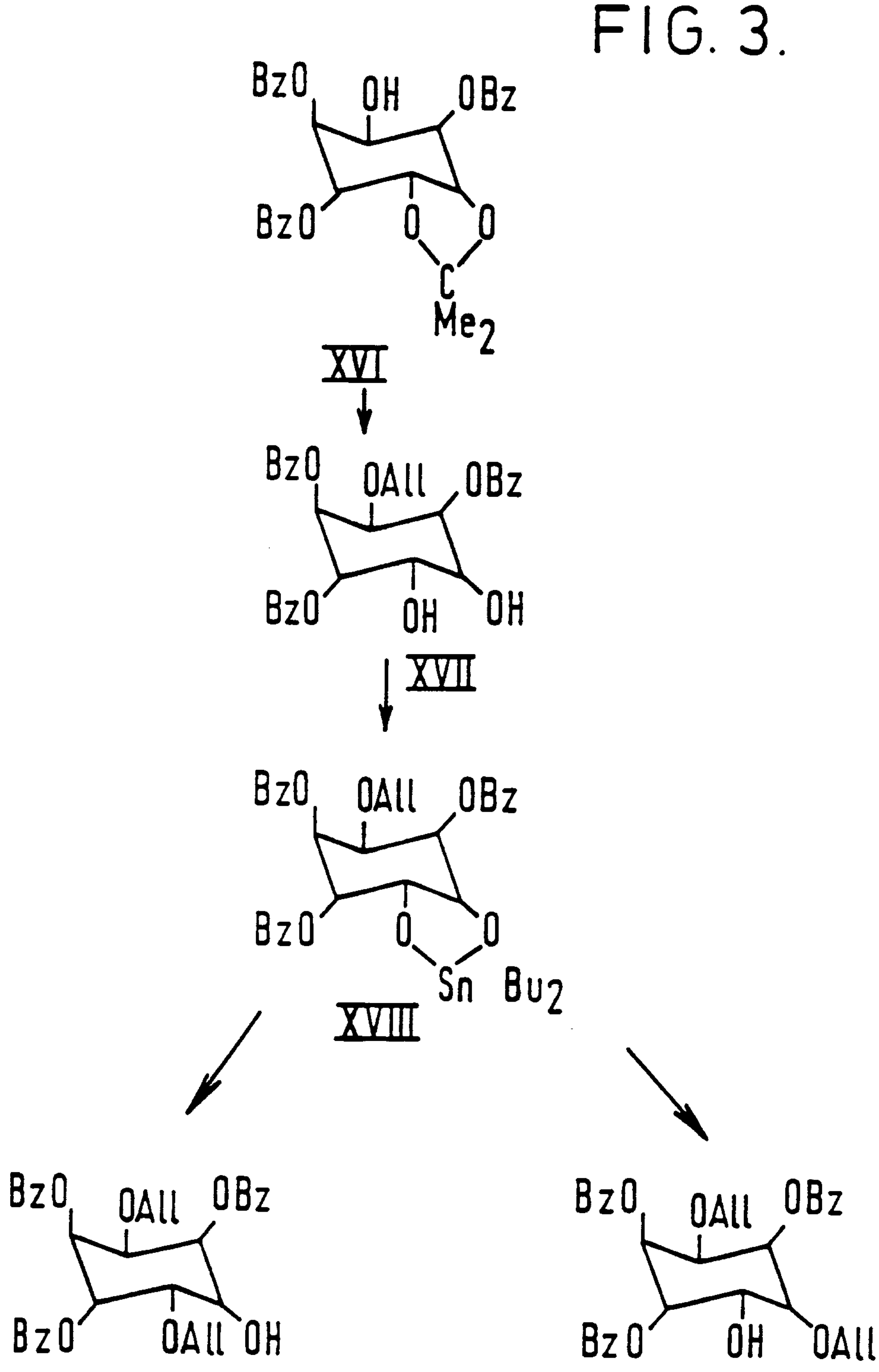

XVI

XVII

XVIII

III

IV

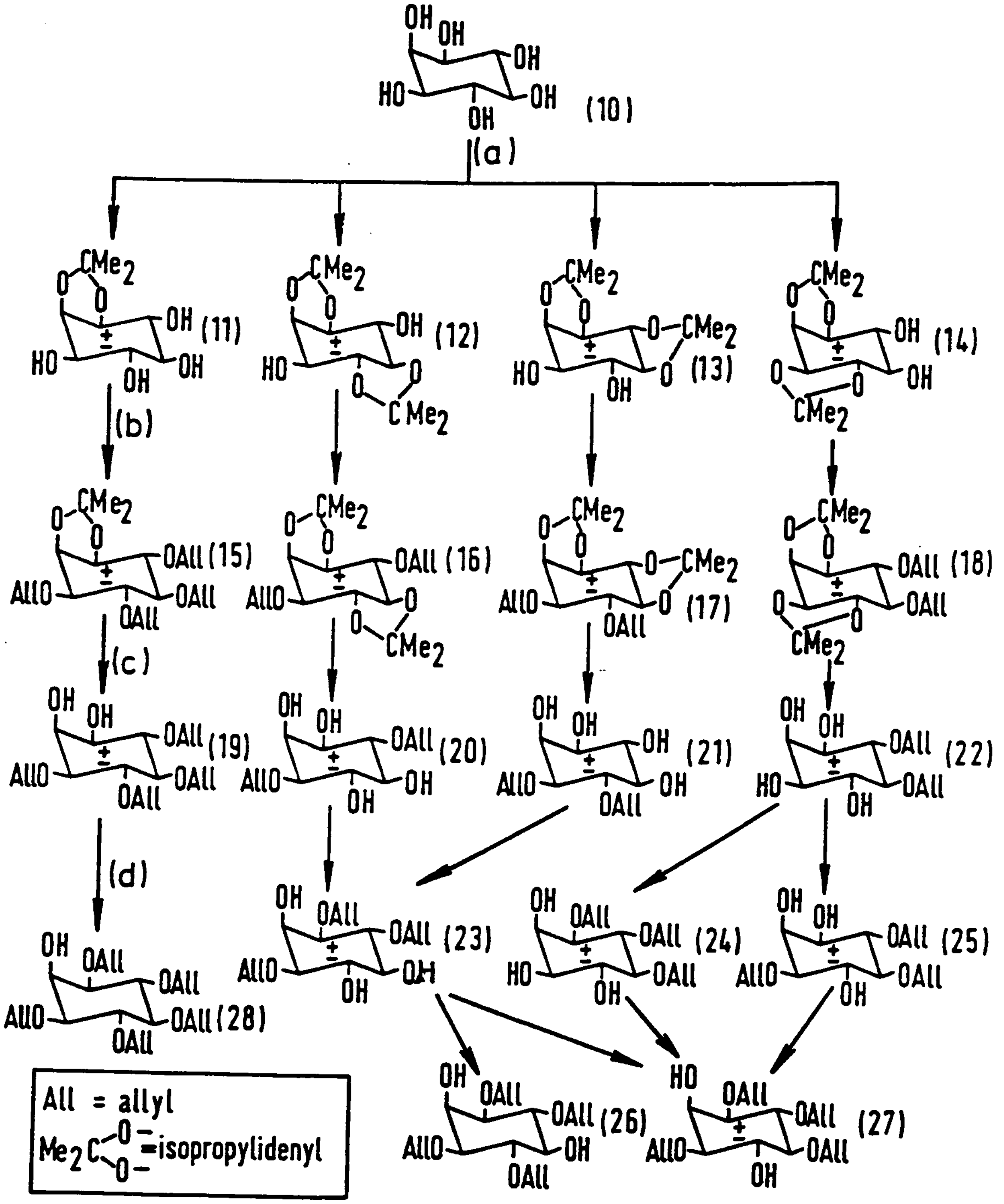
(10)
(a)
(11)
(12)
(13)
(14)
(b)
(15)
(16)
(17)
(18)
(c)
(19)
(20)
(21)
(22)
(d)
(23)
(24)
(25)
(28)
(26)
(27)
All = allyl
=isopropylidenyl

FIG. 4.

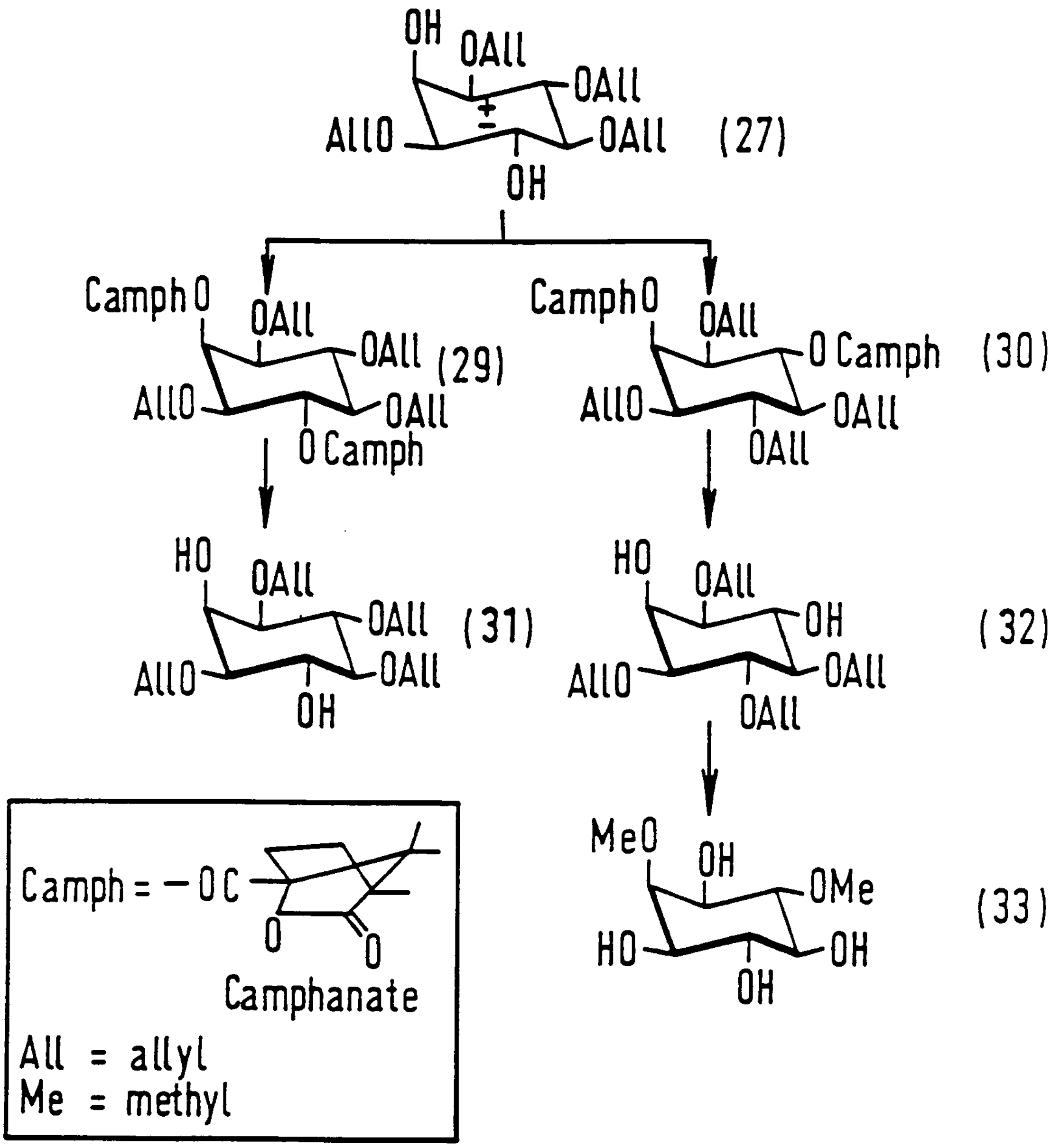


(27)

(29)

(30)

(31)

(32)

(33)

FIG. 5.

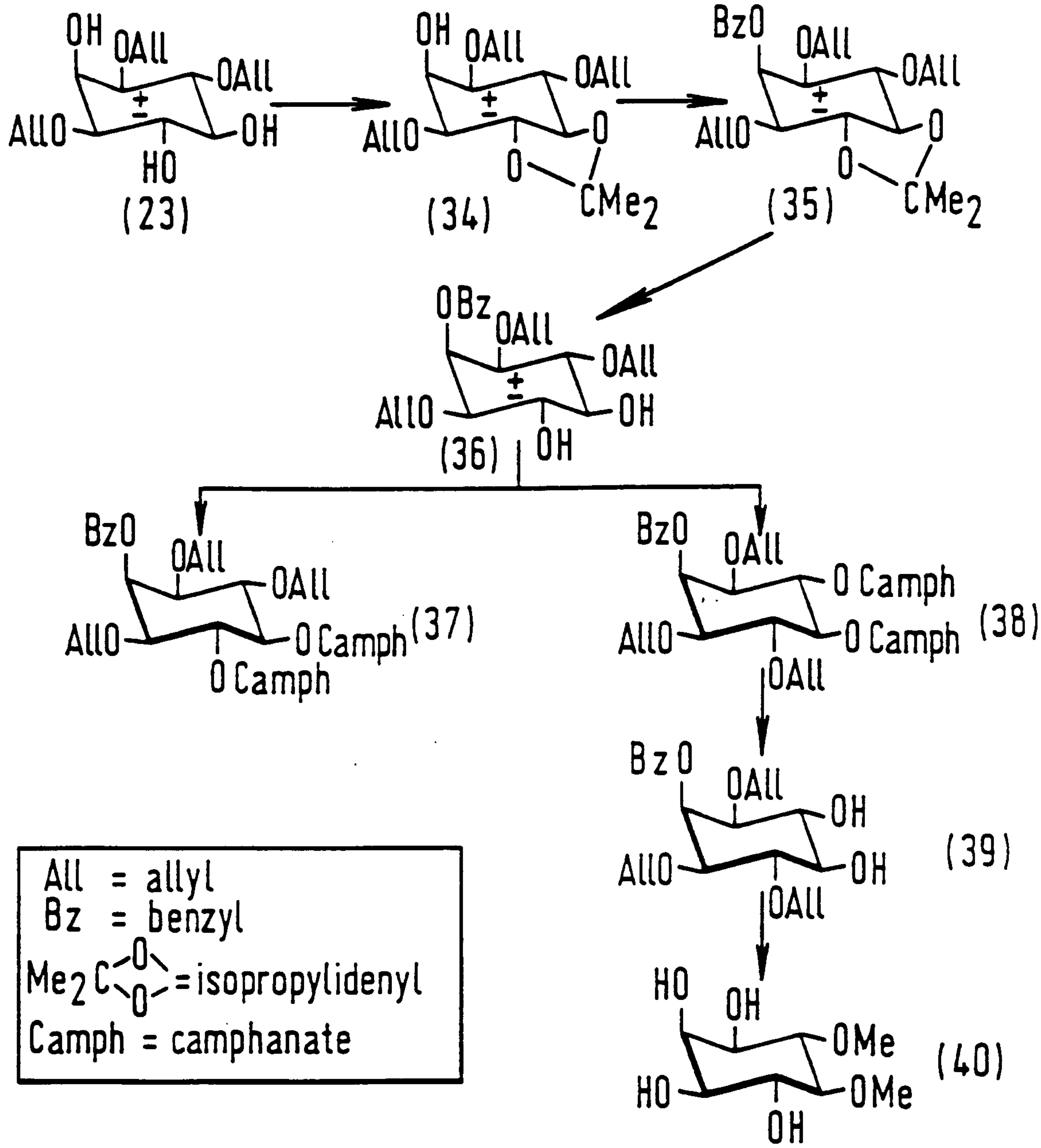


(23) (34) (35) (36) (37) (38) (39) (40)

FIG. 6.

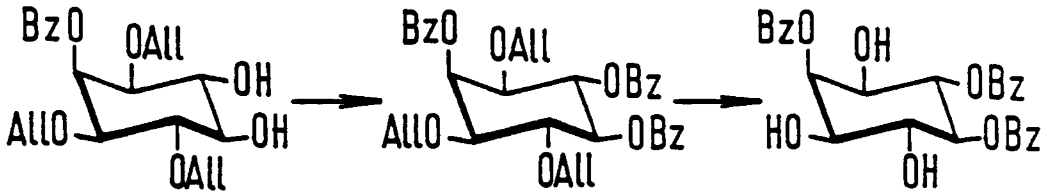

(39) (41) (42)

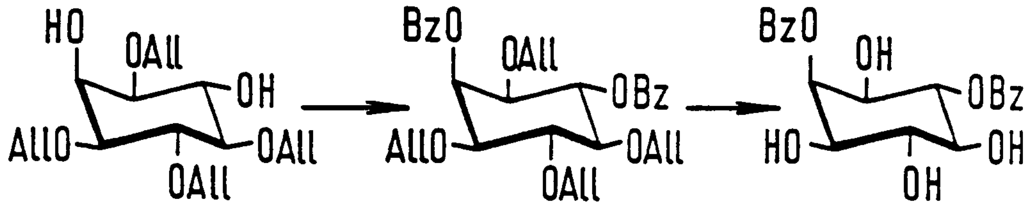

(32) (43) (44)

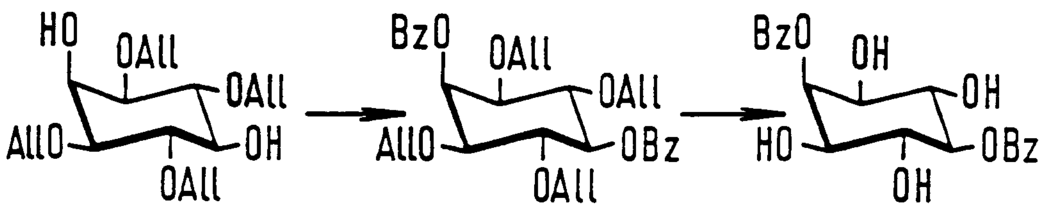

(26) (45) (46)

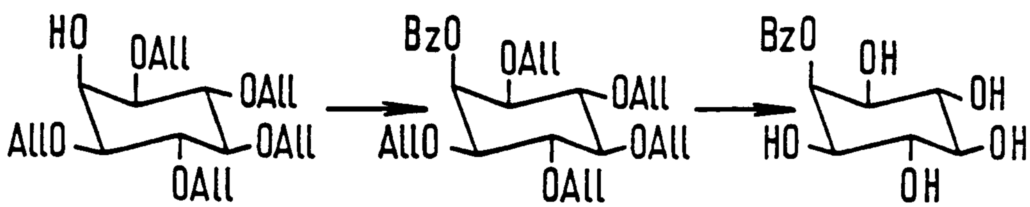

(28) (47) (48)

Bz = benzyl
All = allyl

FIG. 7.

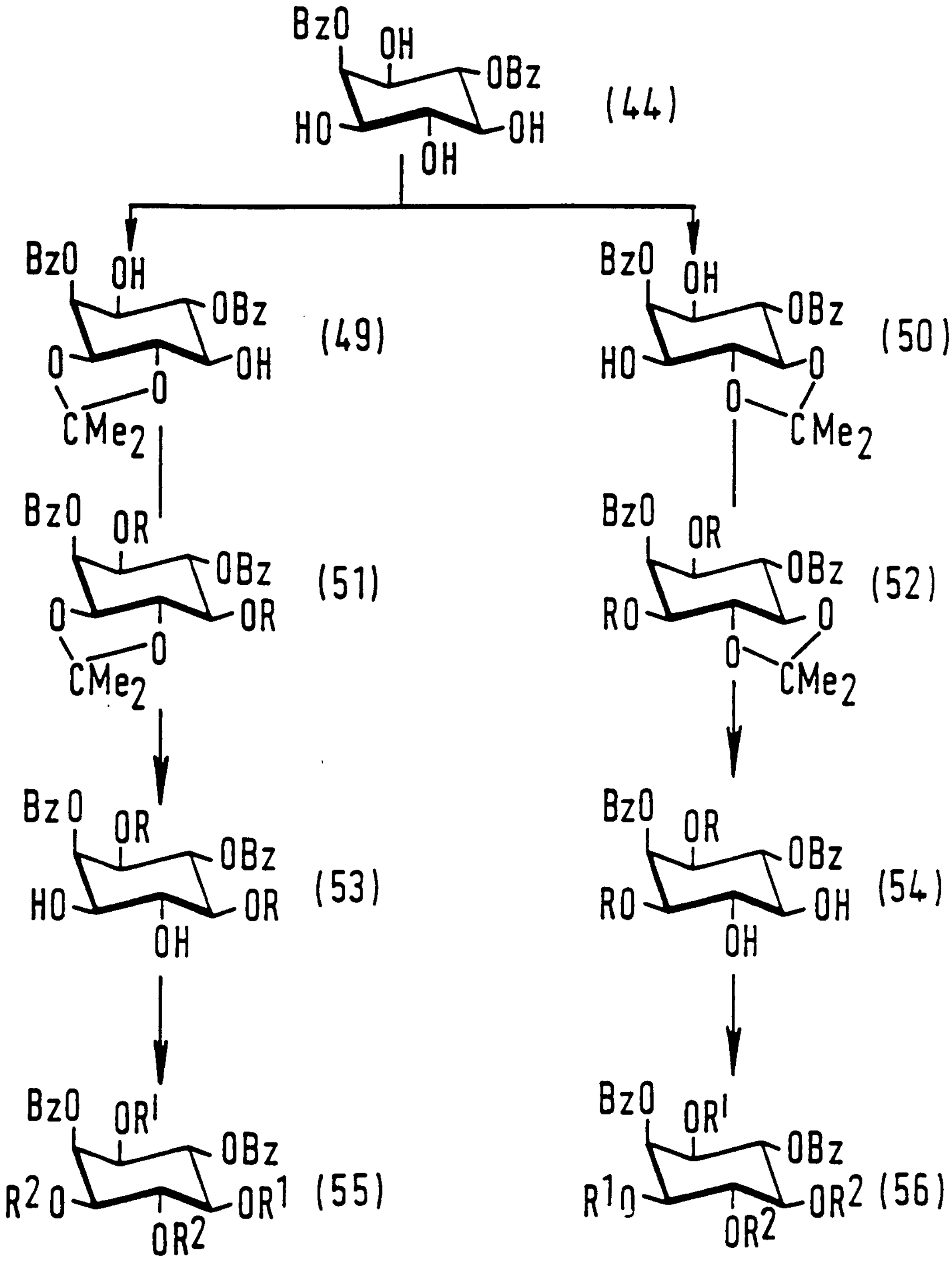

(44)

(49) (50)

(51) (52)

(53) (54)

(55) (56)

R = phosphate or phosphorthioate

FIG. 8.

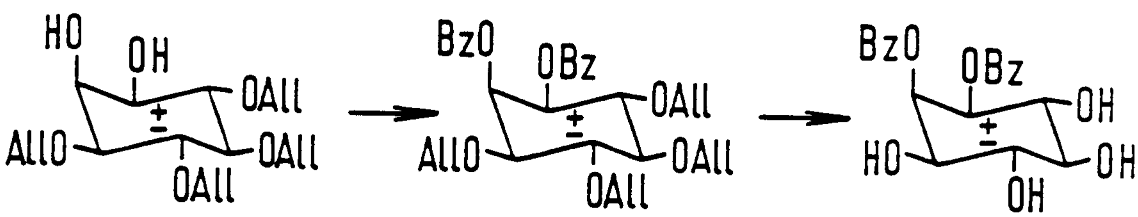

(19) (57) (58)

FIG. 9.

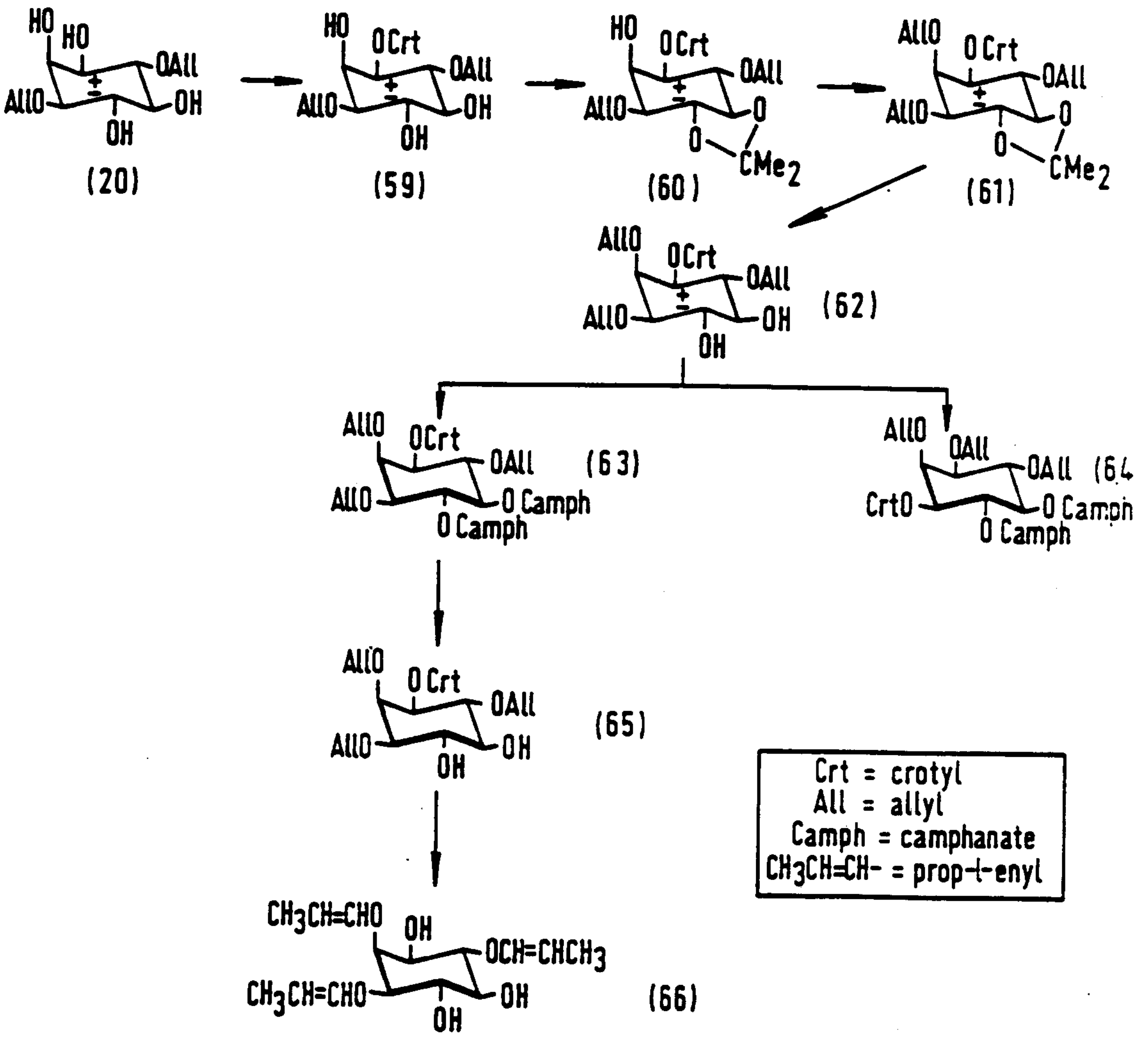

(20) (59) (60) (61) (62) (63) (64) (65) (66)

Crt = crotyl
All = allyl
Camph = camphanate
$CH_3CH$=CH- = prop-1-enyl

FIG. 10.